# EUROPEAN PATENT APPLICATION

(11) **EP 2 253 324 A1**
(43) Date of publication of application: **24.11.2010**
(21) Application number: 09159243.6
(22) Date of filing: 30.04.2009
(51) Int. Cl.: A61K 38/01, C07K 14/47

(54) **Use of a casein-derived peptide and compositions thereof as antihypertensive**

(71) Applicant: CONSEJO SUPERIOR DE INVESTIGACIONES CIENTIFICAS, 28006 Madrid (ES); Universidad de Salamanca, 37008 Salamanca (ES)
(72) Inventor: Recio Sanchez, Isidra, 28006, Madrid (ES); Contreras Gámez, Mar, 28006, Madrid (ES); Amigo Garrido, Lourdes, 28006, Madrid (ES); Ramos González, Mercedes, 28006, Madrid (ES); Montero Gomez, Mª José, 37008, Salamanca (ES); Carrón de la Calle, Rosalía, 37008, Salamanca (ES); Sevilla Toral, Mª Angeles, 37008, Salamanca (ES)
(74) Representative: Pons Ariño, Angel

(57) **Abstract**

Use of a casein-derived peptide with antioxidant and ACE-inhibitory activity *in vitro* and antihypertensive activity in mammals, and compositions thereof. Preferably, this peptide may be use as active ingredient in pharmaceutical, cosmetic and/or dermatological compositions, dietary supplements or as food ingredient.

SEQUENCE LISTING

<110> Consejo Superior de Investigaciones Científicas (CSIC).
Universidad de Salamanca.

<120> Use of a casein-derived peptide and compositions thereof as
antihypertensive.

<130> EP1641.441

<160> 10

<170> PatentIn version 3.4

<210> 1
<211> 5
<212> PRT
<213> Bos sp.

<400> 1

Arg Tyr Leu Gly Tyr
1               5

<210> 2
<211> 8
<212> PRT
<213> Bos sp.

<400> 2

Phe Val Ala Pro Phe Pro Glu Val
1               5

<210> 3
<211> 8
<212> PRT
<213> Bos sp.

<400> 3

Asp Ala Tyr Pro Ser Gly Ala Trp
1               5

<210> 4
<211> 5
<212> PRT
<213> Bos sp.

<400> 4

Asn Leu Leu Arg Phe
1               5

<210> 5
<211> 6
<212> PRT
<213> Bos sp.

<400> 5

Glu Asn Leu Leu Arg Phe
1               5

<210>  6
<211>  8
<212>  PRT
<213>  Bos sp.

<400>  6

Val Gln Val Thr Ser Thr Ala Val
1               5


<210>  7
<211>  6
<212>  PRT
<213>  Bos sp.

<400>  7

Val Thr Ser Thr Ala Val
1               5


<210>  8
<211>  7
<212>  PRT
<213>  Bos sp.

<400>  8

Phe Pro Gln Tyr Leu Gln Tyr
1               5


<210>  9
<211>  7
<212>  PRT
<213>  Bos sp.

<400>  9

Ala Tyr Phe Tyr Pro Glu Leu
1               5


<210>  10
<211>  7
<212>  PRT
<213>  Bos sp.

<400>  10

Tyr Gln Lys Phe Pro Gln Tyr
1               5

## Description

### FIELD OF THE INVENTION

The present invention relates to the use of a casein-derived peptide with antioxidant and ACE-inhibitory activity *in vitro* and antihypertensive activity in mammals, and compositions thereof. Preferably, this peptide may be use as active ingredient in pharmaceutical, cosmetic and/or dermatological compositions, dietary supplements or as food ingredient.

### BACKGROUND OF THE INVENTION

Uncontrolled oxidation processes have been recognized as one of the main causes of food quality decay. These processes are initiated by enzymes such as lipoxygenases (LOX), polyphenol oxidases (PPO) and peroxidases (POX). In order to inhibit such oxidation and peroxidation of unsaturated fatty acids, and to prevent deterioration of product quality, various antioxidants have been conventionally used. Antioxidants (AOX) act on peroxide radicals, which are generated in oxidation, to terminate chain oxidation, or as an alternative act on free radicals to terminate oxidative reaction. Synthetic antioxidants are commonly used antioxidants , such as butylhydroxyanisol (BHA) and butylhydroxytoluene (BHT). However, as their use expands, the safety of synthetic antioxidants has become questioned, since consumers are presenting growing rejections, which results in a decrease of their use. Furthermore, synthetic antioxidants are oil soluble, thus not suitable for their in aqueous solutions.

On the other hand, there are many well known high safety antioxidants such as natural vitamin E (α-tocopherol), vitamin C, and alike are known. However, these natural antioxidants are extremely fat- or water-soluble, so their applications are limited. The natural antioxidants have also disadvantages since their activity cannot be maintained stable for a prolonged period of time.

Furthermore, the intake of peroxides produced form lipid oxidation exerts detrimental effects *in vivo.* Free radicals and active oxygen species generated in the course of oxidation reactions denature proteins in vivo, inactivate enzymes (Szweda et al., 1993 Journal of Biological Chemistry 268: 3342-3347), bring about mutations in DNA (Reiss et al., 1972. Biochemical and Biophysical Research Communication 48:921-926), modify low density lipoproteins (Alaiz et al., 1994. Biological and Pharmaceutical Bulletin 17:51-57), and contribute to aging and various diseases.

The reactive oxygen species (ROS) are implicated inter alia in the cause of carcinogenetic, inflammatory, infectious, cardiovascular and neurological diseases in man and animals (Nair et al., 2003. Mutagenesis 18: 477-483). Antioxidants (AOX) are considered a promising therapeutic approach as they may be playing neuroprotective (preventing apoptosis) and neuroregenerative roles (Moosmann and Behl, 2002. Expert Opinion on Investigational Drugs 11:1407-1435). AOX offer a promising approach in the control or slowing down progression of neurodegegenerative disorders such as Alzheimer's disease, Parkinson's disease, Huntington's disease, amyotrophic lateral sclerosis and ischaemic and haemorrhagic stroke (Maxwell, 1995; Floyd, 1999. Proceedings of the Society for Experimental Biology and Medicine 222:235-245; Mattson 2000. Nature Rev. Mol. Cell Biol. 1: 120-130; Moosmann and Behl, 2002. Expert Opinion on Investigational Drugs 11:1407-1435; Nair et al., 2003. Mutagenesis 18: 477-483; Berger 2005. Clinical Nutrition 24:172-183). The most important exogenous AOX are dietary phytochemicals (such as polyphenols, quinones, flavonoids, catechins, coumarins, terpenoids) and the smaller molecules like ascorbic acid (Vitamin C), alpha-tocopherol (Vitamin E) and beta-carotene vitamin E. Because of the importance in preventing oxidation to biological processes and to improved stability of products subject to oxidation, there remains a need to identify new antioxidative compounds, such as antioxidant peptides.

Dietary antioxidants may also help to prevent cardiovascular diseases (Krinksy 1993. Annual review of nutrition 13: 561-587); Parthasarathy, 1998. Journal of Medicinal Food 1:45-51). Cardiovascular diseases represent the first cause of morbidity and mortality in Western countries, with hypertension affecting about 20% of the world adult population (Miguel et al., 2007. Food Chemistry 104: 163-168). High blood pressure is a common risk factor for cardiovascular diseases, and more accurately, this disease induces cerebrovascular incidents, heart failure and kidney disease, which all could lead to more complicated dysfunctions of the internal organs.

Although most of the mechanisms associated with hypertension are unknown, is well studied that one regulator of hypertension, the angiotensin I-converting enzyme (ACE), which is a zinc-containing dipeptidylcarboxypeptidase (EC 3.4.15.1), plays an important physiological role in regulating blood pressure (Skeggs et al., 1956. The Journal of Experimental Medicine 103: 295-299). The greater the ACE activity, the more angiotensin I is converted to angiotensin II, which induces high blood pressure. ACE converts an inactive form of the decapeptide, angiotensin I, to a potent vasoconstrictor, the octapeptide angiotensin II, and also inactivates bradykinin which reduces blood pressure. For these reasons, specific inhibitors of ACE are useful for regulating physiological activities associated with ACE in the human body (Ondetti et al., 1977. Science 196: 441-444).

Potent synthetic ACE inhibitors such as captopril, enalapril, alacepril, lisinopril and ramipril are widely used in the clinical treatment of hypertension and heart disorders in humans. These synthetic drugs, however, have several side effects including coughing, taste disturbances and skin rashes. Food-derived ACE inhibitors have safety advantages over the synthetic compounds (Atkinson & Robertson 1979. Lancet 2: 836-839). As a large number of individuals suffer from such disease, scientists believe that other methods rather than chemical and pharmacological medication should be identified in the effort to reduce hypertensive diseases. Therefore, potential biological, functional and nutraceutical methods should be utilized as a treatment to minimize the number of individuals who fall into the hypertensive category and have been afflicted with diseases arising from this.

Some peptides have recently been reported to play an important role in controlling the development of hypertension by regulating the rennin angiotensin system (Arihara et al., 2001. Meat Science, 57: 319-324; Asihda et al., 1996. Clinical and Experimental Hypertension 18: 851-871; Haws et al., 1994. Pediatric Research 35: 671-676.; Katayama et al., 2004. Journal of Agricultural and Food Chemistry 52: 771-775; Katayama et al., 2007. Journal of Food Science 72: S702-S706; Katayama et al., 2008. Journal of Agricultural and Food Chemistry 56: 355-360). There are of course other studies concerned with ACE inhibitory peptides with a view to enhance the value of food products. Many of these peptides have been sourced from animal products such as bovine casein, porcine muscle, egg white, yak milk casein, sheep milk yoghurt and oyster (Miguel et al., 2009, Food Chemistry 112: 211-214; Miguel and Fidel, 2007. Food Chemistry 102: 511-515; Miguel et al., 2007. Food Chemistry 104: 163-168; Maoa et al., 2007. Food Chemistry 103: 1282-1287; Papadimitriou et al., 2007. Food Chemistry 105: 647-656; and Wang et al., 2008. Food Chemistry 111: 302-308, respectively). Some have been found to be effective for antihypertensive agents in vivo without causing side effects in spontaneously hypertensive rats (SHR) and hypertensive humans (Li et al., 2004. Nutrition Research 27:469-486).

Milk proteins (mainly α-lactalbumin, caseins, β-lactoglobulin, immunoglobulins, lactoferrin and serum albumin) are known for having a wide range of nutritional, functional and biological properties that make them important ingredients in functional or health promoting foods. These properties are partly attributed to bioactive peptides coded in the different milk proteins. At present, numerous peptides exhibiting various activities, such as opiate, antithrombotic and antioxidant activities, cholesterol-lowering ability, antimicrobial, immunodulatory, mineral carrier and antihypertensive activity have been reported. Moreover, some peptides are multifunctional and can exert more than one of the effects mentioned. Bioactive peptides are often inactive within the sequence of the parent protein and can be released by enzymatic hydrolysis.

Powerful ACE inhibitory hydrolysates were obtained from whey protein after hydrolysis with trypsin (Ferreira et al., 2007. International Dairy Journal 17: 481-487; Mullally et al., 1997. International Dairy Journal 7: 299-303) and α-chymotrypsin (Da Costaa et al., 2007. International Dairy Journal 17: 632-640). Digestive enzymes have also been employed for the release of antihypertensive peptides from the milk casein fraction, for instance, this is the case of trypsin or pepsin (López-Expósito et al., 2007. Le Lait 87: 241-249; Miguel et al., 2009. Food Chemistry 112: 211-214). Spectrophotometric, fluorimetric, chromatographic and capillary electrophoresis techniques have been used to isolate the active peptides and to measure their ability to inhibit ACE in vitro. However, it is only through *in vivo* studies that the antihypertensive effects of a given hydrolysate or peptide can be reliably assessed. Several studies have been performed to determine the antihypertensive effects of ACE-inhibitory peptides using spontaneously hypertensive rats (SHR) (Miguel et al., 2005. British Journal of Nutrition 94: 731-737; Muguerza et al., 2006. International Dairy Journal, 16: 61-69; Nakamura et al., 1995. Journal of Dairy Science 78: 1253-1257; for a review see, FitzGerald et al., 2004. Journal of Nutrition 134: 980S-988S; López-Fandiño et al., 2006. International Dairy Journal 16:1277-1293), but only few milk-derived peptides have been tested for their antihypertensive effect in humans (Hata et al., 1996. American Journal of Clinical Nutrition 64: 767-771; Mizuno et al., 2005. British Journal of Nutrition 94: 84-91; Jauhiainen et al., 2005. American Journal of Hypertension 18: 1600-1605; Jauhiainen et al., 2007. International Dairy Journal 17: 1209-1211; Seppo et al., 2003. American Journal of Clinical Nutrition 77: 326-330).

The IC₅₀ value (inhibitor concentration leading to 50% inhibition) is used to estimate the effectiveness of different ACE inhibitory peptides. However, it is not always directly related to the *in vivo* hypotensive effect. Some peptides can be susceptible to degradation or modification in the gut, the vascular system and the liver. Being an inhibitor of ACE is not enough for a peptide to be able to reduce hypertension in a hypertensive subject; it should also be bioavailable in order to reach intact the renin-angiotensin mechanism of the smooth muscles of blood vessel walls. There are several examples of peptides, which showing a potent ACE-inhibitory activity, did not exert antihypertensive effect *in vivo.* For instance, peptide αs1-casein f(23-27) that showed a potent ACE-inhibitory activity but no hypotensive effect in SHR (Maruyama et al., 1987. Agricultural and Biological Chemistry 51: 2557-2561), and peptide β-lactoglobulin f(142-148) with in vitro ACE-inhibitory activity but did not have effect in human volunteers (Walsh et al., 2004. Journal of Dairy Science 87: 3845-3857).

As high blood pressure can lead to damaging effects on the brain, heart and blood vessels, it is essential to reduce the incidence of this condition among the population. Given the important role of diet in the prevention and treatment of hypertension, and that is now demonstrated that the consumption of food products containing antihypertensive peptides produces a significantly reduction in blood pressure (Jauhiainen & Korpela, 2007. Journal of Nutrition 137: 825S-829S) it is necessary to identified peptides with antihypertensive activity *in vivo* that can be used as active ingredients in pharmaceutical and food compositions.

### DETAILED DESCRIPTION OF THE INVENTION

The present invention provides a casein-derived peptide with antioxidant and ACE-inhibitory activity *in vitro* and antihypertensive activity in mammals.

In one aspect the invention relates to an isolated peptide comprising the amino acid sequence of SEQ ID NO: 1, hereinafter peptide of the invention, for use as antihypertensive. In a preferred embodiment of this aspect of the invention, the amino acid sequence of the peptide has no more than 10 amino acids. In another preferred embodiment the amino acid sequence of the peptide has no more than 8 amino acids. In another preferred embodiment of this aspect of the invention, the amino acid sequence of the peptide consists of SEQ ID NO: 1. In a more preferred embodiment, the mammal is a human.

Hypertension, also referred to as high blood pressure, HTN or HPN, is a medical condition in which the blood pressure is chronically elevated. In current usage, the word "hypertension" without a qualifier normally refers to systemic, arterial hypertension. In individuals older than 50 years, hypertension is considered to be present when a person's blood pressure is consistently at least 140 mm Hg systolic or 90 mm Hg diastolic. Persistent hypertension is one of the risk factors for strokes, heart attacks, heart failure and arterial aneurysm, and is a leading cause of chronic renal failure. Even moderate elevation of arterial blood pressure leads to shortened life expectancy. At severely high pressures, defined as mean arterial pressures 50% or more above average, a person can expect to live no more than a few years unless appropriately treated. Beginning at a systolic pressure of 115 mm Hg and diastolic pressure of 75 mm Hg (commonly written as 115/75 mm Hg), cardiovascular disease (CVD) risk doubles for each increment of 20/10 mm Hg (Chobanian et al., 2003. Hypertension 42 (6): 1206-1252).

The term "isolated" herein, as used in "isolated peptide," refers to material, such as a nucleic acid, a peptide or a protein, which is (1) substantially or essentially free from components that normally accompany or interact with it as found in its naturally occurring environment. The isolated material optionally comprises material not found which the material in its natural environment, or (2) if the material is in its natural environment, the material has been synthetically (non naturally) altered by deliberate human intervention.

Another aspect of the invention relates to the peptide of the invention, comprising the amino acid sequence of SEQ ID NO: 1, for use as a medicament, or to the use of the peptide of the invention for the manufacture of a medicament.

In a preferred embodiment of this aspect of the invention, the amino acid sequence of the peptide has no more than 10 amino acids. In another preferred embodiment the amino acid sequence of the peptide has no more than 8 amino acids. In another preferred embodiment of this aspect of the invention, the amino acid sequence of the peptide consists of SEQ ID NO: 1. In a more preferred embodiment, the mammal is a human.

Another aspect of the invention relates to the peptide of the invention, comprising the amino acid sequence of SEQ ID NO: 1, for use in the treatment or prevention of hypertension in a mammal including a human, or to the use of the peptide of the invention for the manufacture of a medicament for treatment of hypertension in a mammal including a human. In a preferred embodiment of this aspect of the invention, the amino acid sequence of the peptide has no more than 10 amino acids. In another preferred embodiment the amino acid sequence of the peptide has no more than 8 amino acids. In another preferred embodiment of this aspect of the invention, the amino acid sequence of the peptide consists of SEQ ID NO: 1. In a more preferred embodiment, the mammal is a human.

Another aspect of the invention relates to the peptide of the invention, comprising the amino acid sequence of SEQ ID NO: 1, for use in the treatment or prevention of a disease involving oxidation in a mammal including a human, or to the use of the peptide of the invention for the manufacture of a medicament for treatment of a disease involving oxidation in a mammal including a human. In a preferred embodiment of this aspect of the invention, the amino acid sequence of the peptide has no more than 10 amino acids. In another preferred embodiment the amino acid sequence of the peptide has no more than 8 amino acids. In another preferred embodiment of this aspect of the invention, the amino acid sequence of the peptide consists of SEQ ID NO: 1. In a more preferred embodiment, the mammal is a human. In another preferred embodiment, the disease involving oxidation is selected from the list comprising: osteoarthritis, rheumatoid arthritis, ischemia, cataract, corneal pathology, glaucoma, retinal degeneration, vitreal degeneration, cancer, immune deficiency, hyperimmunity, autoimmunity, neurodegeneration, aging, Alzheimer's disease, Huntington's disease, Machado-Joseph disease, multiple sclerosis, muscular dystrophy, Parkinson's disease, senility, muscular atrophy, stroke, hepatopathies, systemic lupus erythematosus, mixed connective tissue disease, multiple sclerosis, and diabetes.

The peptide of the invention having an ability to reduce the hypertension in a mammal, could also show an ACE-inhibitory or antioxidative activity per se, or should be hydrolysed in order to release the bioactive peptide. As is know by a person having ordinary skill in the art, the peptide of the invention could be like an acid or base addition salt, in particular pharmaceutically acceptable salts, esters, solvates and anhydrates. The term "pharmaceutically acceptable salts" or "solvates" refers to any pharmaceutically acceptable salt, ester, solvate, or any other compound which, upon administration to the recipient is capable of providing (directly or indirectly) a peptide as described herein. However, it will be appreciated that non-pharmaceutically acceptable salts also fall within the scope of the invention since those may be useful in the preparation of pharmaceutically acceptable salts. The preparation of salts and derivatives can be carried out by methods known in the art.

The peptide of the invention can be administered in a substantial pure form to a person or can be given as part of a more complex composition. When the peptide is given to a person in need thereof as part of a more complex mixture, the amount of this mixture given to this person should be such that the peptide mentioned in this invention reaches the effective amount. Examples of mixtures that contain this peptide or to which the peptide might be added are dried protein extracts, hydrolyzed protein extracts, dried plant extracts, cacao powder, a dried food composition, etc. A person in need of the mentioned peptide, can consume the peptide of this invention in a pure form as a pharmaceutical composition, as part of a composition containing this peptide, for instance as a dietary supplement, or as part of a food matrix whereto this peptide has been added in an effective amount. Examples of food matrixes to which an anti-hypertensive and/or antioxidative composition containing the peptide of this invention can be added include, but are not limited to: beverages, infused foods, milk, yogurts, cheese, fermented milk, flavoured milk drinks, soybean milk, precooked cereals, bread, cake, butter, margarine, sauces, frying oils, vegetable oils, corn oil, olive oil, soybean oil, palm oil, safflower oil, olive oil, sunflower oil, cottonseed oil, condiments, salad dressings, fruit juices, syrups, desserts, icings and fillings, soft frozen products, confections, chewing gum and intermediate food.

Then, another preferred aspect of the invention relates to a composition, hereinafter first composition of the invention, comprising the peptide of the invention. In a preferred embodiment of this aspect of the invention, the amino acid sequence of the peptide has no more than 10 amino acids. In another preferred embodiment of this aspect of the invention, the amino acid sequence of the peptide consists of SEQ ID NO: 1. In another preferred embodiment of this aspect of the invention, the mammal is a human.

In a further preferred embodiment, the first composition of the invention consists in a food composition. The food composition comprises the peptide of the invention in an effective amount to reduce hyperthension in mammals and/or to reduce the excess reactive oxygen species (ROS) in a mammal, including a human. Preferred food compositions are selected from: a beverage, infused food, milk, yogurt, cheese, fermented milk, flavoured milk drink, soybean milk, precooked cereals, bread, cake, butter, margarine, sauces, frying oils, vegetable oils, corn oil, olive oil, soybean oil, palm oil, safflower oil, olive oil, sunflower oil, cottonseed oil, condiments, salad dressings, fruit juices, syrups, desserts, icings and fillings, soft frozen products, confections, chewing gum and intermediate food. Also within the food compositions, the present invention contemplates a protein hydrolysate, obtained from mammal milk, enriched in the peptide of the invention or derivatives thereof. In a preferred embodiment the protein hydrolysate is obtained from bovine milk. In a preferred protein hydrolysate according to the invention, the amount of the peptide of the invention or salts thereof is between 0.005% and 3%, preferably between 0,01% and 1% of the protein hydrolysate. It is clear that the use of the peptide and derivatives thereof, as well as the protein hydrolysates of the invention can be employed in the preparation of food compositions, which include dietary supplements and food ingredients. In another preferred embodiment the nutritional or dietary supplement comprises a sterile composition containing the peptide of invention, preferably provided with a gastric juice resistant release delaying coating. In another preferred embodiment the food composition, including the protein hydrolysate and the nutritional or dietary supplement comprises appropriate "carriers" such as diluents, adjuvants, excipients, or vehicles with which the peptide is administered. Suitable appropriate excipients include, but are not limited to starch, glucose, fructose, lactose, sucrose, gelatin, malt, rice, flour, chalk, silica gel, sodium stearate, glycerol monostearate, talc, sodium chloride, dried skim milk, glycerol, propylene, glycol, water, ethanol, and the like. Such nutritional supplements may be used to combat a wide range of conditions associated with hypertension and/or with the excess reactive oxygen species, and they help maintain health or a healthy lifestyle to the mammal, preferably human. The food composition also has properties for preventing hypertension in mammals, preferably human beings.

In a further preferred embodiment, the first composition of the invention is a pharmaceutical composition comprising the peptide of the invention or a pharmaceutically acceptable derivative thereof and a pharmaceutically acceptable carrier, binder and/or auxiliary material. Said peptide or derivative thereof is preferably present in the composition in an effective amount to reduce hypertension in mammals. The carrier materials, binders and/or auxiliary materials must be pharmaceutically and pharmacologically tolerable, so that they can be combined with the other components of the formulation or preparation and do not exert adverse effects on the organism treated. The pharmaceutical compositions can be in the form of single doses. The compositions are prepared according to methods known in the field of pharmacology. The appropriate quantities of active substances suitable for administration may vary as a function of the particularly field therapy. Generally, the active substance concentration in a single-dose formulation is 1% to 95% of the total formulation. Preferably, the peptide of the invention is orally administered to a mammal in a dose above 2-3 mg/day. The compositions of the invention may conveniently comprise the peptide of the invention incorporated into a solid, liquid or aqueous carrier medium and the pharmaceutical compositions may be in forms suitable for topical, enteral, oral, rectal, vaginal, or parenteral (e.g. intravenous, subcutaneous, intramuscular or intravascular) administration to the mammal, and preferably human body. The term "carrier" refers to a diluent, adjuvant, excipient, or vehicle with which the therapeutic is administered. Such pharmaceutical carriers can be sterile liquids, such as water and oils, including those of petroleum, animal, vegetable, or synthetic origin, such as peanut oil, soybean oil, mineral oil, sesame oil, and the like. Suitable pharmaceutical excipients include starch, glucose, fructose, lactose, sucrose, gelatin, malt, rice, flour, chalk, silica gel, sodium stearate, glycerol monostearate, talc, sodium chloride, dried skim milk, glycerol, propylene, glycol, water, ethanol, and the like. The compositions of the present invention are however especially suited to the oral administration. For oral administration, the pharmaceutical compositions of the invention may be formulated using conventional pharmaceutical carriers and excipients, e.g. in the form of tablets, coated tablets, syrups, capsules, pills, powders, suspensions, emulsions, sprays, etc. The pharmaceutical compositions of the invention may of course contain further ingredients, such as for example conventional pharmaceutical formulation aids, e.g. emulsifiers, extenders, flavours, colouring agents, surfactants, pH adjusting agents, ointment bases, geling agents, propellants, stabilizers and the like. in a preferred embodiment the pharmaceutical compositions may also contain other physiologically active agents or therapeutic ingredients. It will be understood that the therapeutic dose of each active ingredient administered in accordance with the present invention will vary depending upon the particular active ingredient employed (including the peptide of the invention), the mode by which the active ingredient is to be administered, and the condition or disorder to be treated.

A further aspect the invention relates to the first composition of the invention for use as a medicament, or to the use of the first composition of the invention for the manufacture of a medicament. A preferred embodiment of this aspect of the invention, relates to the first composition of the invention for use in the treatment or prophylaxis of hypertension, stroke, coronary disease, myocardial infarction, metabolic syndrome, peripheral vascular disease or abdominal aortic aneurysm in mammals including a human, or to the use of the first composition of the invention in the manufacture of a medicament for the treatment or prophylaxis of hypertension, stroke, coronary disease, myocardial infarction, metabolic syndrome, peripheral vascular disease or abdominal aortic aneurysm in mammals including a human. Another preferred embodiment of this aspect of the invention, relates to the first composition of the invention for use in the treatment of a disease involving oxidation in mammals including a human, or to the use of the first composition of the invention in the manufacture of a medicament for the treatment or prophylaxis of a disease involving oxidation in mammals including a human. In another preferred embodiment of this aspect of the invention, the disease involving oxidation is selected from the list comprising: osteoarthritis, rheumatoid arthritis, ischemia, cataract, corneal pathology, glaucoma, retinal degeneration, vitreal degeneration, cancer, immune deficiency, hyperimmunity, autoimmunity, neurodegeneration, aging, Alzheimer's disease, Huntington's disease, Machado-Joseph disease, multiple sclerosis, muscular dystrophy, Parkinson's disease, senility, muscular atrophy, stroke, hepatopathies, systemic lupus erythematosus, mixed connective tissue disease, multiple sclerosis, and diabetes.

As used herein, "mammal" includes any organism within class *Mammalia,* superrclass *Gnathostomata,* subphylum *Craniata,* phylum *Chordata,* kingdom *Metazoa,* and superkingdom *Eukaryota.*

In the context of the present specification, the term "medicament" refers to any substance or combination of substances which may be used in or administered to mammals or human beings either with a view to restoring, correcting or modifying physiological functions by exerting a pharmacological, immunological or metabolic action, or to making a medical diagnosis.

As defined here, an "active substance", "pharmaceutically active substance", "active ingredient", "active pharmaceutical ingredient" or "therapeutic ingredient" refers to any substance or mixture of substances that are intended to furnish pharmacological activity or other direct effect in the diagnosis, cure, mitigation, treatment, or prevention of disease or medical condition, or to affect the structure and the function of the body. The peptide of the invention is a bioactive peptide, a natural or functional ingredient with antihypertensive activity.

The term "bioactive peptide" refers to any peptide that has biological activity. In particular, such bioactivity includes a capacity to bind to a receptor molecule, or a capacity to enhance or impair enzyme catalysis, cellular signaling, transcription, translation, or other cellular processes. The term "peptide" refers to a molecule having at least two amino acid residues bonded together through one or more peptide bond(s).

Another aspect of the invention relates to the use of a peptide comprising the amino acid sequence of SEQ ID NO: 1, hereinafter peptide of the invention, as antioxidant. In one embodiment, the amino acid sequence consisting essentially of the amino acid sequence of SEQ ID NO:1. In another embodiment, the amino acid sequence consisting of the amino acid sequence of SEQ ID NO: 1.

Articles such as food, cosmetics, and medicines, that contain oils and fats, suffer from oxidation or peroxidation of the oil and fat components caused by atmospheric oxygen, which is the most serious problem in storage, preservation, and processing of such articles. Unsaturated fatty acids contained in oils and fats, such as linoleic and linolenic acids, are known to be particularly prone to peroxidation by oxygen to generate lipid peroxides, free radicals, or even carcinogenic substances. Oxidation and peroxidation cause not only coloration, discoloration, denaturalization, abnormal odor, or decrease in effective nutritional value of the articles, but also generation of toxic substances, which results in deterioration of product quality. In another aspect, the current invention is a composition, hereinafter second composition of the invention, comprising:
a) a product ingredient subject to oxidation; and
b) an antioxidative peptide comprising an amino acid sequence of SEQ ID NO:1 present in an amount effective for reducing oxidation of the product ingredient subject to oxidation.

"Effective for reducing" means that the reduction in oxidation observed with samples treated with the antioxidative peptide of the current invention is statistically significant when compared to the results using controls (i.e., samples without addition of the antioxidative peptide of the invention). This statistical significance can be calculated and determined by methods well known in the art. For example, statistical significance can be determined by utilizing a T-test and a 90%, or preferably a 95%, probability cut-off value.

Then, the second composition of the invention could be an stabilized product. As used herein "stabilized" products are products that are resistant to oxidative stress that occurs upon product storage over time. This resistance to oxidative stress may result in increased maximum storage time, shelf-life, or expiration dating, or increased consistency of taste and/or other organoleptic properties over time, as compared to products containing the same product ingredient subject to oxidation without the antioxidative peptides of the current invention.

Product ingredients subject to oxidation typically include products that are foods, cosmetics, pharmaceuticals, or medical diagnostics. However, the present invention can include any commercial product ingredient that is subject to oxidation. In one embodiment of this aspect of the invention, the product ingredient subject to oxidation is a food ingredient and the product is a food. In another embodiment, the product ingredient subject to oxidation is a cosmetic component and the product is a cosmetic. In another embodiment, the product ingredient subject to oxidation is a medical diagnostic component and the product is a medical diagnostic. In another embodiment, the product ingredient subject to oxidation is an active ingredient, active substance, or a pharmaceutically active substance, and the product is a pharmaceutical product, a pharmaceutical composition and/or a medicament.

In the present invention, all of the technical and scientific terms are of the same meaning as that commonly understood by an expert in the field to which the invention pertains. Throughout the description and the claims, the word "comprises" and the variations thereof are not intended to exclude other technical features, components or steps. For the person skilled in the art, other objects, advantages and characteristics of the invention will be implied in part from the description and in part from the practice of the invention. The following examples and drawings are provided for illustrative purposes and are not intended to be limiting of the present invention.

### DESCRIPTION OF THE DRAWINGS

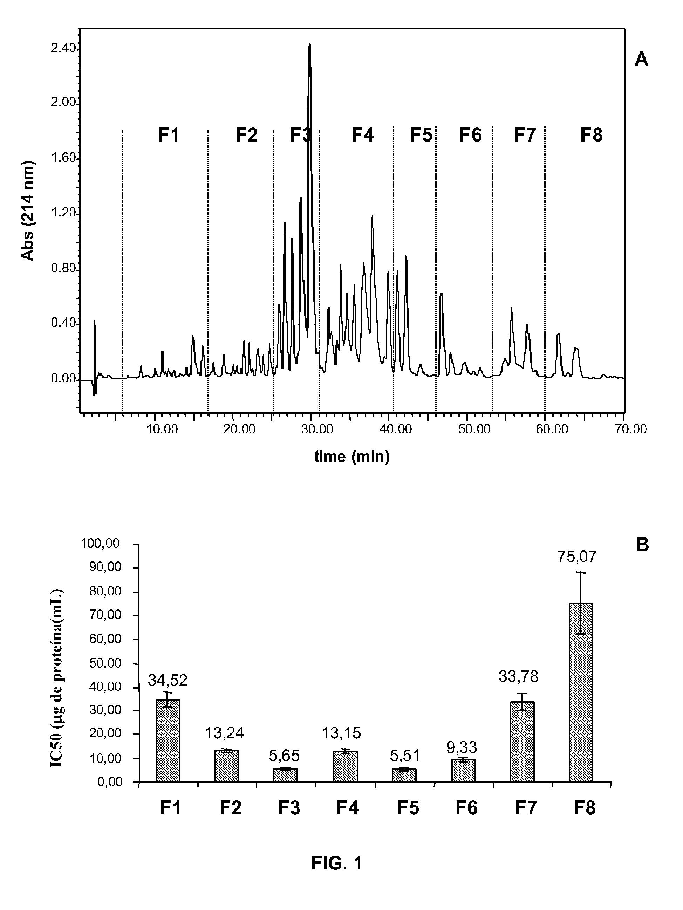

**Fig. 1.** (A) Fractionation by RP-HPLC at semi-preparative scale of the 3000 Da-permeate obtained from the peptic hydrolysate of bovine casein. Collected fractions are termed with F followed with a number (F1-F8). (B) Angiotensin-converting enzyme-inhibitory activity, expressed as IC₅₀ value, of the collected fractions from the semi-preparative RP-HPLC system. For the IC₅₀ determination purposes, protein content of the fractions collected from the HPLC was estimated by the bicinchoninic acid assay. Data are expressed as mean ± SEM for a minimum of three experiments.

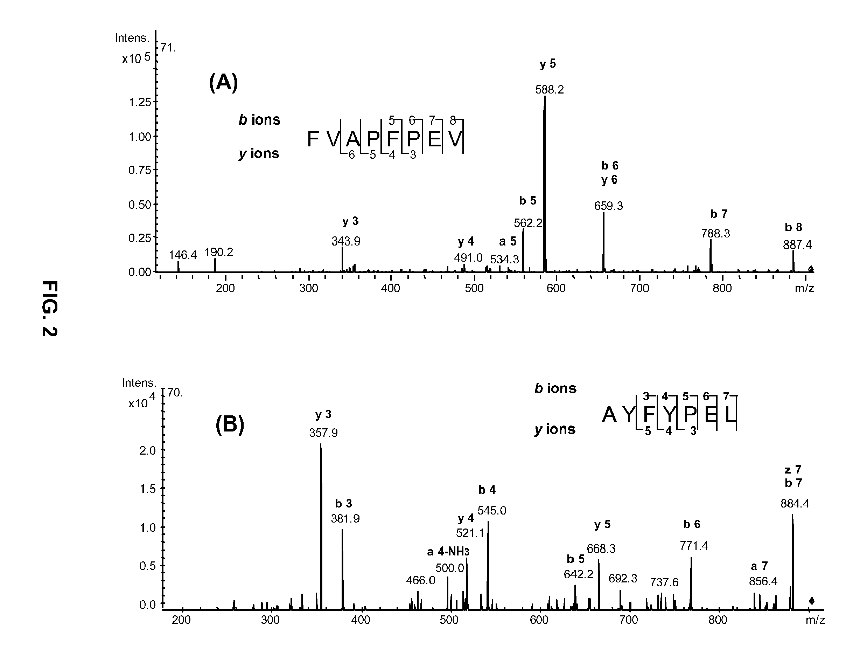

**Fig. 2.** (A) Tandem mass spectrum of the singly charged ion m/z 905.5 and (B) of singly charged ion m/z 902.5, both included in fraction 5. Following sequence interpretation and data base searching, the peptides were identified as αₛ₁-casein f(24-31) and αₛ₁-casein f(143-149), respectively. The sequences of these peptides are displayed with the fragment ions observed in the spectra.

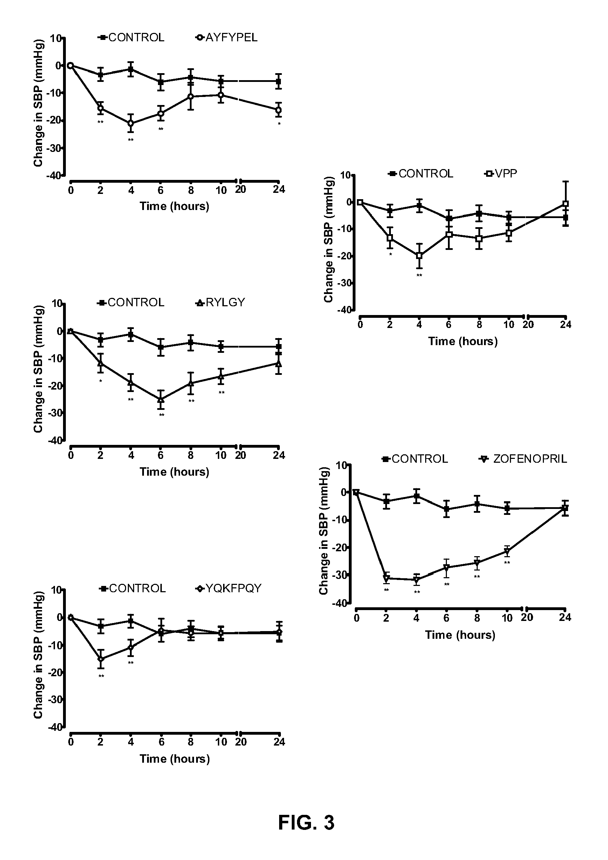

**Fig. 3****.** Change in systolic blood pressure (SBP) after oral administration of different peptides (5 mg kg⁻¹). Data are expressed as mean ± SEM for a minimum of four animals (n=4-8). Control groups received the same volume of water. Unpaired student's t-test was used to compare groups, **p*< 0.05, ***p*< 0.01 vs control group.

Below are examples that illustrate this invention:

### EXAMPLE

### Production of Casein Hydrolysates

A 0.5% (w/v) aqueous solution of isoelectric casein was adjusted to pH 2.0 2.5 with 1 M HCI and digested with 3.7% (w/w of substrate) porcine pepsin A (Sigma, St. Louis, MO, USA) for 24 h at 37°C in an orbital shaker at 150 rpm. Pepsin was added at the beginning and after 4 h of hydrolysis. Aliquots of the hydrolysate were taken during hydrolysis at 1, 2, 3, 4, 5, 6, 7 and 24 h. The reaction was terminated by heating at 80°C for 15 min and the pH was adjusted to 7.0 by addition of 1 M NaOH. The hydrolysates were centrifuged at 16.000 x g for 15 min and the supernatants removed. The protein concentration of hydrolysates was determined by Kjeldahl method and the ACE-inhibitory activity was tested.

### Isolation of ACE-Inhibitory Peptides from the Peptic Hydrolysate

The 3-h peptic hydrolysate was subjected to ultrafiltration through a hydrophilic 3000 Da cut-off membrane (Millipore Corporation, Bedford, MA, USA) with a stirred ultrafiltration cell (model 8400, Millipore). The permeate from this ultrafiltration step was subjected to semipreparative RP-HPLC on a Waters Series 600 HPLC, equipped with Millennium 3.2 Software for data acquisition (Waters Corporation, Mildford, MA, USA) as described in Quirós et al., 2007 (Dairy Journal 17: 33-41), but elution was performed with a linear gradient of solvent B in A from 0% to 21 % B in 30 min, 21 % to 35% in 40 min and to 35% to 70% in 5 min at 30°C and a flow rate of 4 mL min-1. Solvent A was a mixture of water-trifluoroacetic acid (1000:1) and solvent B contained a mixture of acetonitrile-trifluoroacetic acid (1000:0.8). Fractions from the HPLC system were freeze-dried and kept at -20°C until use.

### Peptide sequencing by RP-HPLC-MS/MS

RP-HPLC-MS/MS analysis of the active fractions was performed on an Agilent 1100 HPLC System (Agilent Technologies, Waldbron, Germany) connected on-line to an Esquire 3000 quadrupole ion trap (Bruker Daltonik GmbH, Bremen, Germany) equipped with an electrospray ionisation source, as described in Hernández-Ledesma et al., 2005 (Journal of Agriculture and Food Chemistry 53: 588-593).

### Measurement of ACE-Inhibitory Activity

ACE-inhibitory activity was measured by Cushman & Cheung 1971 (Biochemical Pharmacology, 20, 1637-1648) spectrophotometric assay with some modifications, as reported by (Quirós et al., 2007). For this purpose, protein concentration of the aliquots of the pepsin hydrolysate was determined by the Kjeldahl method. The protein content of the fractions collected from HPLC was estimated by the bicinchoninic acid assay (Pierce, Rockford, IL, USA) using bovine serum albumin, as standard, and peptide concentration of the chemically synthesized peptides was based in the dry weight of the peptides. Chemical synthesized peptides were purchased from GenScript Corp. (Piscataway, NJ, USA).

### Measurement of Antioxidant Activity

The ORAC-FL assay was based on the method proposed by Ou et al. 2001 (Journal of Agriculture and Food Chemistry, 49, 4619-4626) and modified by Dávalos et al. 2004 (Journal of Agriculture and Food Chemistry, 52, 48-54).

### Measurement of Blood Pressure

All procedures were carried out in accordance with conventional guidelines for experimentation with animals. Twelve-week old male SHR rats were used (Janvier, France); they were housed in groups of four per cage in a regulated environment with a 12 h light/dark cycle in a standard experimental laboratory of the Animal Experimentation Service of the Salamanca University (N°PAE SA001). The animals were deprived of solid diet 12 hours before experiments began but received a nutritive solution of 8% sucrose in 0.2% NaCl to avoid excessive dehydratation. The rats received a single dose of the synthesized peptides or zofenopril (5 mg kg⁻¹) dissolved in ultrapure water and the control group received the same volume of water. Systolic blood pressure (SBP) was measured in awake rats with an automated multichannel system, using the tail-cuff method with a photoelectric sensor (Niprem 546, Cibertec SA, Spain) as described in Guerrero et al., 2003 (Journal of Cardiovascular Pharmacology 42: 348-355). SBP was measured before administration, and also at 2, 4, 6, 8, 10 and 24 hours post-administration. The changes in SBP were expressed as the differences before and after administration of the different products. Unpaired student's t-test was used to compare groups.

### Results

### Evolution of ACE-Inhibitory Activity during Casein Hydrolysis

The progress of the hydrolysis reaction of casein with pepsin was monitored by taking samples at different intervals. The water-soluble fractions of the hydrolysates were analyzed by HPLC-MS and the ACE-inhibitory activity and protein content were determined. As shown in Table 1, hydrolysis of caseins with pepsin under our conditions occurred rapidly, giving a protein content in the soluble fraction of 0.45% (w/v) after 1 hour of hydrolysis. The ACE-inhibitory activity of the soluble fraction increased during the first 3 hours of hydrolysis and the lowest value for the IC₅₀ value (22.19 µg mL⁻¹), i.e., the maximum activity, was reached after 3 hours hydrolysis. This IC₅₀ value was comparable to the activity found in other previously reported antihypertensive protein hydrolysates obtained by enzymatic digestion or by fermentation (Miguel et al., 2004. Journal of Food Protection 67: 1914-1920; Muguerza et al., 2006. International Dairy Journal 16: 61-69).

**Table 1: Angiotensin-converting enzyme-inhibitory activity, expressed as the protein concentration required to inhibit the enzyme activity by 50% (IC₅₀) of the water-soluble fraction from the casein hydrolysates prepared with pepsin**

| Hydrolysis time | IC₅₀^{a} (µg protein/mL) | Protein (% w/v) |
|---|---|---|
| 1 h | 60.85 ± 10.8 | 0.45 |
| 2 h | 35.27 ± 3.6 | 0.45 |
| 3 h | 22.19 ± 1.6 | 0.45 |
| 4 h | 29.65 ± 3.9 | 0.46 |
| 5 h | 38.85 ± 3.7 | 0.47 |
| 6 h | 45.48 ± 10.1 | 0.46 |
| 7 h | 37.07 ± 2.8 | 0.47 |
| 24 h | 25.68 ± 1.6 | 0.47 |

### Identification of ACE-Inhibitory Peptides

The water-soluble fraction of the 3h-hydrolysate with pepsin was first subjected to ultrafiltration through a 3000 Da cut-off membrane and the ACE-inhibitory activity of the permeate and the retentate was measured. The activity of the 3000 Da permeate (IC₅₀ 5.68 ± 183 0.36 µg mL⁻¹) was four times higher than that found in the total water-soluble extract (IC₅₀ 22.19 ± 1.61 µg mL⁻¹) and 40 times higher than the activity measured in the retentate fraction (IC₅₀ 231 ± 35 µg mL-1).

The 3000 Da permeate was subjected to semi-preparative RP-HPLC. The 70-min chromatogram was divided into 8 fractions, which were collected, concentrated by freeze drying, and then their ACE-inhibitory activity was measured (Fig. 1A and 1B). Two fractions, F3 and F5 in Fig. 1, exhibited the highest ACE-inhibitory activities, with IC₅₀ values of 5.65± 0.54 µg mL⁻¹ and 5.51± 0.60 µg mL⁻¹. The other three chromatographic fractions, F2, F4 and F6, also showed notable potent ACE-inhibitory activity with IC₅₀ values under 14 µg mL^{-1.} All these active chromatographic fractions (F2, F3, F4, F5 and F6 in Fig. 1 were analysed by HPLC-MS/MS. As an example, Fig. 2A shows the MS/MS spectrum of the two most abundant ions in the active fraction, F5. The fragmentation spectrum of ion m/z 905.5 matched with the αₛ₁-CN-derived peptide f(21-31) with sequence SEQ ID NO: 2. This spectrum contained a major ion at m/z 588.2, which was identified as a y-type fragment ion resulting from the cleavage N-terminal to proline (y5). It is known that the presence of proline residues in a peptide has a significant effect upon the fragments observed by MS/MS, and the fragment ions-produced N-terminal to proline are over-represented in the spectra. The fragment ions corresponding to the fragmentation N-terminal to the other proline residue, i.e., b5 and y3 occurred at lower abundance than y5 although similar X-P relative bond cleavage ratios have been reported when X was alanine or phenylalanine (Breci et al., 2003. Analytical Chemistry 75: 1963-1971). The MS/MS spectrum in Fig. 2B was also consistent with the sequence AYFYPEL that corresponds to fragment (143-149) from αₛ₁-CN. In this spectrum, the presence of proline residues and its unusual fragmentation patterns helped identification, being fragment ions *y*3 and *b*4 (corresponding to cleavage N-terminal to proline), the most prominent fragment ions.

The major peptide components of each active HPLC fraction were identified and these results are summarised in Table 2. A total of 44 peptide sequences were identified of which 13 peptides corresponded to β-casein (β-CN) fragments, and 19 to αₛ₁-CN fragments. The most abundant peptides in each chromatographic fraction are in bold in Table 2. Among the identified peptides, one of them had previously been described as an ACE inhibitor. More specifically, the αₛ₁-CN peptide SEQ ID NO: 3, had been found to exhibit ACE-inhibitory activity with an IC₅₀ value of 98 µM (Pihlanto-Leppälä et al., 1998. International Dairy Journal 8: 325-331). Other peptides show high homology with ACE-inhibitory peptides previously described in the literature, such as, SEQ ID NO: 4 which share five residues with SEQ ID NO: 5, and have an IC₅₀ value of 82.4 µM (Quirós et al., 2005. Journal of Dairy Science 88: 3480-3487). Similarly, peptide SEQ ID NO: 6 includes the sequence SEQ ID NO: 7 (IC₅₀, 52 µM) (NZ508867 A and WO9965326), and peptide LQY, found in fraction 2, corresponds to the C218 terminal tripeptide of SEQ ID O: 8 (IC₅₀, 14 µM) (Tauzin et al., 2002. FEBS Letters 531:369-374). However, the presence of these peptides with moderate ACE-inhibitory activity could not explain the high activity found in the permeate from the casein hydrolysate and in the isolated fractions most abundant peptides in each chromatographic fraction are in bold in Table 2.

### In vitro ACE-Inhibitory and Antioxidant Activity

In an attempt to identify the peptides responsible for the ACE-inhibitory activity in the 3000 Da permeate from the casein hydrolysate, several peptides, among the most abundant in each chromatographic fraction were selected on the basis of their sequences, especially the C226 terminal tripeptide. A total of six peptides were chemically synthesized and the ACE227 inhibitory, calculated as IC₅₀ value, was measured (Table 3). Because it has been reported that antioxidant activity of several antihypertensive peptides can also contribute to their activity (Duffy et al., 1999. The Lancet 354: 2048-2049), the radical scavenging activity of these synthetic sequences was also evaluated. Three of the six peptides, with sequences SEQ ID NO: 1, SEQ ID NO: 9 and SEQ ID NO: 10, exhibited potent ACE-inhibitory activity with IC₅₀ values lower than 20 µM (Table 3). Interestingly, these three peptides also had high radical scavenging activity with values at least two times higher than Trolox. The two αₛ₁-casein-derived peptides, i.e., SEQ ID NO: 1 and SEQ ID NO: 9, were among the most abundant in the hydrolysate and the 3000 Da-permeate fraction obtained from it, and therefore, given their activity (IC₅₀ values of 0.71 and 6.58 µM, respectively) and concentration, they might be responsible for the activity found in the total hydrolysate.

**Table 2: Identification of peptides contained in the RP-HPLC fractions with angiotensin-converting enzyme-inhibitory activity.**

| **Fraction^{a}** | **Ion (m/z) for MS/MS (charge)** | **Calculated mass** | **Observed mass^{b}** | **Protein Fragment** | **Sequence^{c}** |
|---|---|---|---|---|---|
| **F2** | 675.1(1) | 674.32 | 674.1 | β-CN f(1-5) | RELEE |
| | 795.2(1) | 794.35 | 794.2 | β-CN f(118-124) | PFTESQS |
| | 763.2(1) | 762.36 | 762.2 | α_{S1}-CN f(56-61) | DIKQME |
| | 423.1(1) | 422.22 | 422.1 | α_{S2-}CN f(96-98) | LQY |
| | 804.3(1) | 803.44 | 803.3 | κ-CN f(162-169) | VQVTSTAV |
| | 521.2(1) | 520.27 | 520.2 | α_{S2}-CN f(85-88) | INQF |
| | 628.3(1) | 627.32 | 627.3 | β-CN f(6-11) | LNVPGE |
| | 502.2(1) | 501.28 | 501.2 | α_{S1}-CN f(95-98) | LEQL |
| | 423.2(1) | 422.22 | 422.2 | α_{S1}-CN f(154- | YQL |
| **F3** | 597.5(1) | 596.31 | 596.5 | 156) α_{S1}-CN f(150- | **FRQF** |
| | 671.5(1) | 670.34 | 670.5 | 153) α_{S1}-CN f(90-94) | **RYLGY** |
| | 973.5(1) | 972.47 | 972.5 | α_{S2}-CN f(89-95) | YQKFPQY |
| | 800.4(1) | 799.34 | 799.4 | α_{S1}-CN f(173-179) | **YTDAPSF** |
| | 866.4(1) | 865.37 | 865.4 | α_{S1}-CN f(157-164) | **DAYPSGAW** |
| | 940.5(1) | 939.48 | 939.5 | α_{S1}-CN f(165-172) | **YYVPLGTQ** |
| | 465.3(1) | 464.26 | 464.3 | α_{S1}-CN f(92-95) | LGYL |
| | 890.5(1) | 889.48 | 889.5 | κ-CN f(138-146) | AVESTVATL |
| **F4** | 787.3(1) | 786.43 | 786.3 | β-CN f(157-163) | **FPPQSVL** |
| | 784.3(1) | 783.43 | 783.3 | α_{S1}-CN f(90-95) | **RYLGYL** |
| | 1001.3(1) | 1000.51 | 1000.3 | α_{S1}-CN f(150-156) | **FRQFYQL** |
| | 662.3(1) | 661.39 | 661.3 | α_{S1}-CN f(19-23) | **NLLRF** |
| | 831.2(1) | 830.39 | 830.2 | α_{S1}-CN f(144-149) | **YFYPEL** |
| | 1198.4(1) | 1197.62 | 1197.4 | κ-CN f(96-105) | ARHPHPHLSF |
| **F5** | 957.4(2) | 1912.91 | 1912.8 | β-CN f(141-156) | QSWMHQPHQPLPPTVM |
| | 1422.8(1) | 1421.79 | 1421.8 | κ-CN f(18-29) | FSDKIAKYIPIQ |
| | 617.4(1) | 617.32 | 616.4 | κ-CN f(53-57) | NQFLP |
| | 1219.6(1) | 1218.63 | 1218.6 | α_{S1}-CN f(25-35) | VAPFPEVFGKE |
| | 729.5(1) | 728.40 | 728.5 | β-CN f(199-205) | GPVRGPF |
| | 902.5(1) | 901.42 | 901.5 | α_{S1}-CN f(143-149) | AYFYPEL |
| | 845.6(1) | 844.44 | 844.6 | κ-CN f(51-57) | **INNQFLP** |
| | 905.5(1) | 904.47 | 904.5 | α_{S1}-CN f(24-31) | **FVAPFPEV** |
| | 1063.4(2) | 2124.12 | 2124.0 | κ-CN f(51-68) | INNQFLPYPYYAKPAAVR |
| **F6** | 919.4(2) | 1835.95 | 1836.8 | α_{S1}-CN f(24-39) | FVAPFPEVFGKEKVNE |
| | 1215.6(2) | 2429.27 | 2429.2 | β-CN f(59-80) | VYPFPGPIHNSLPQNIPPLTQT |
| | 1151.7(1) | 1150.69 | 1150.7 | β-CN f(199-209) | GPVRGPFPIIV |
| | 1094.7(1) | 1093.66 | 1093.7 | β-CN f(200-209) | PVRGPFPIIV |
| | 802.6(1) | 801.51 | 801.6 | β-CN f(134-140) | HLPLPLL |
| | 905.5(1) | 904.47 | 904.5 | α_{S1}-CN f(25-32) | VAPFPEVF |
| | 995.6(1) | 994.59 | 994.6 | β-CN f(81-89) | PVVVPPFLQ |
| | 1300.6(1) | 1299.69 | 1299.6 | β-CNA² f(59-70) | VYPFPGPIPNSL |
| | 1008.6(1) | 1007.58 | 1007.6 | κ-CN f(25-32) | YIPIQYVL |
| | 527.2(1) | 526.35 | 526.2 | κ-CN f(46-50) | KPVAL |
| | 742.6(1) | 741.44 | 741.6 | β-CN f(203-209) | GPFPIIV |
| | 1015.5(1) | 1014.51 | 1014.5 | α_{S1}-CN f(142-149) | LAYFYPEL |

| | | | | | |
|---|---|---|---|---|---|
| ^{a} Fractions are termed as in Fig. 1 ^{b} Calculated monoisotopic mass ^{c} Amino acids are designed using one letter code. Most abundant peptides in each fraction are in bold. | | | | | |

### Antihypertensive Activity of the Synthetic Peptides

The antihypertensive effect of the peptides with higher ACE-inhibitory potency was examined in SHR. Animals used for the experiment had an average basal SBP of 178±4 mmHg. Fig. 3 shows the time-course changes in SBP after oral administration of the synthetic peptides and after the administration of zofenopril (positive control). All the peptides displayed antihypertensive effects. Peptide SEQ ID NO: 1 lowered SBP in a progressive and maintained manner during most of the time of study. The maximum reduction, observed 6 hours post-administration, was 25±3 mmHg (Fig. 3). Peptide SEQ ID NO: 9 showed a significant effect but a smaller and less maintained effect than this reached with the same dose of SEQ ID NO: 1. A small and short effect was observed with SEQ ID NO: 10. The maximum antihypertensive effect of VPP (20±5 mmHg) was similar to that of SEQ ID NO: 9, but none of the peptides reached the decrease in SBP caused by zofenopril (Fig. 3).

**Table 3: Angiotensin-converting enzyme-inhibitory activity and radical scavenging activity of synthetic peptides selected from those identified in the active chromatographic fractions.**

| Sequence | Protein fragment | IC₅₀^{a}± SE (µM) | ORAC-FL± SEM^{b} (µmol equiv Trolox/µmol peptide) |
|---|---|---|---|
| RYLGY | α_{S1}-CN f(90-94) | 0.71 ± 0.08 | 2.829 ± 0.040 |
| AYFYPEL | α_{S1}-CN f(143-149) | 6.58 ± 0.50 | 3.216 ± 0.114 |
| YQKFPQY | α_{S2}-CN f(89-95) | 20.08 ± 1.25 | 2.033 ± 0.077 |
| HLPLPLL | β-CN f(134-140) | 34.40 ± 2.22 | 0.060 ± 0.002 |
| VAPFPEVF | α_{S1}-CN f(25-32) | 362.50 ± 23.56 | 0.046 ± 0.001 |
| FVAPFPEV | α_{S1}-CN f(24-31) | 475.89 ± 41.73 | <0.025 |

| | | | |
|---|---|---|---|
| ^{a} IC₅₀ value corresponds to the concentration of peptide needed to inhibit 50% of the original ACE activity, expressed as mean ± standard error (n= 3) ^{b} Standard error of the mean (n=3) | | | |

## Claims

1. An isolated peptide comprising the amino acid sequence of SEQ ID NO: 1, for use as a medicament.

2. An isolated peptide comprising the amino acid sequence of SEQ ID NO: 1, for use in the treatment or prevention of hypertension in a mammal including a human.

3. The peptide according to claim 2, for use in the treatment or prevention of stroke, coronary disease, myocardial infarction, metabolic syndrome, peripheral vascular disease or abdominal aortic aneurysm in mammals including a human.

4. The peptide according to any of claims 1-3, having no more than 10 amino acids.

5. The peptide according to any of claims 1-3, having no more than 8 amino acids.

6. The peptide according to any of claims 1-5, wherein the amino acid sequence consists of SEQ ID NO: 1.

7. The peptide according to any of claims 1-6, wherein the mammal is a human.

8. A composition comprising a peptide as described in any of claims 1-7.

9. The composition according to claim 8, wherein the composition is a food composition.

10. The food composition according to claim 9, wherein the composition comprises a protein hydrolysate.

11. The food composition according to claim 10, wherein the protein hydrolysate is obtained from bovine milk.

12. The food composition according to any of claims 9-11, wherein the amount of the peptide as described in any of claims 1-7 or salts thereof is between 0.005% and 3%.

13. A composition according to claim 8, wherein the composition is a pharmaceutical composition.

14. The pharmaceutical composition according to claim 13, additionally comprising a pharmaceutically acceptable carrier.

15. The pharmaceutical composition according to any of claims 13-14, additionally comprising another therapeutic ingredient.
